**Europäisches Patentamt**

**European Patent Office**

**Office européen des brevets**

(19)

(11) Numéro de publication: **0 221 942 B1**

(12) **FASCICULE DE BREVET EUROPEEN**

(45) Date de publication du fascicule du brevet: **16.08.89**

(51) Int. Cl.⁴: **B 01 D 53/34**, C 07 C 148/04, C 07 C 9/04, C 10 K 1/34

(21) Numéro de dépôt: **86902852.2**

(22) Date de dépôt: **06.05.86**

(86) Numéro de dépôt international: **PCT/FR 86/00157**

(87) Numéro de publication internationale: **WO 86/06649** (20.11.86 Gazette 86/25)

(54) **PROCEDE REGENERATIF POUR L'ELIMINATION DES MERCAPTANS CONTENUS DANS UN GAZ.**

(30) Priorité: **10.05.85 FR 8507121**

(43) Date de publication de la demande: **20.05.87 Bulletin 87/21**

(45) Mention de la délivrance du brevet: **16.08.89 Bulletin 89/33**

(84) Etats contractants désignés: **BE DE FR GB IT NL**

(56) Documents cités: **GB-A- 1 078 828**

**Chemical Abstracts, vol. 73, 1970, Columbus, Ohio, (US); A.U. Strunina et al.: "Removal of mercaptans from natural gaz by active carbon", p. 115, résumé 100679d**

(73) Titulaire: **SOCIETE NATIONALE ELF AQUITAINE (PRODUCTION)**, Tour Elf 2, Place de la Coupole La Défense 6, F-92400 Courbevoie (FR)

(72) Inventeur: **VOIRIN, Robert**, Lotissement des Cascades, Rue Daniel Argote F-64300 Orthez (FR)
Inventeur: **ELGUE, Jean**, 1, chemin Clohare, Lons F-64140 Billère (FR)

(74) Mandataire: **Boillot, Marc**, SOCIETE NATIONALE ELF AQUITAINE Division Propriété Industrielle Tour Elf, F-92078 Paris la Défense Cédex 45 (FR)

## Description

L'invention concerne un procédé régénératif pour l'élimination des mercaptans contenus dans un gaz en faisant appel à un absorbant à base d'au moins un oxyde métallique actif, c'est-à-dire susceptible de retenir les mercaptans par réaction chimique. Elle se rapporte plus particulièrement à un perfectionnement à la régénération de l'absorbant ayant fixé les mercaptans.

On sait que des gaz d'origines diverses, notamment gaz naturels gaz de synthèse ou encore gaz résiduaires, renferment, à titre d'impuretés, de faibles concentrations de mercaptans et qu'il est nécessaire d'éliminer ces mercaptans du fait de leur odeur extrêmement désagréable et surtout de leur toxicité, avant d'acheminer lesdits gaz soit vers les circuits d'utilisation de ces gaz lorsqu'il s'agit notamment de gaz naturels ou de gaz de synthèse ou encore vers l'atmosphère dans le cas de gaz résiduaires.

Parmi les procédés proposés pour éliminer les mercaptans contenus dans un gaz, on connaît un procédé régénératif faisant appel à un absorbant solide à base d'un oxyde métallique actif tel que l'oxyde cuivrique pour retenir les mercaptans par formation de mercaptides (FR-A-2 119 803). Un tel procédé est particulièrement adapté pour le traitement de gaz naturels renfermant des mercaptans car il n'est pas perturbé par la présence d'hydrocarbures.

Ce procédé régénératif comporte une étape d'absorption au cours de laquelle on met en contact le gaz à traiter avec l'absorbant solide, à des températures inférieures à 100°C, pour retenir les mercaptans sur l'absorbant. L'absorbant chargé des composés soufrés qu'il a retenu est soumis après quelques cycles d'absorption à une réactivation et lorsque la réactivation ne permet plus de conférer à l'absorbant une activité suffisante, on fait subir une régénération à l'absorbant désactivé.

Pour effectuer la réactivation de l'absorbant, on balaie cet absorbant, dans une première phase, avec un gaz renfermant une proportion majoritaire d'hydrogène ainsi qu'une faible quantité d'ammoniac en opérant entre 200°C et 250°C et, dans une deuxième phase, en vue de réoxyder ledit absorbant, avec de l'air en opérant entre 100°C et 200°C.

La régénération de l'absorbant est une opération plus complexe au cours de laquelle ledit absorbant, après avoir été balayé rapidement par un courant d'azote pour chasser les hydrocarbures qu'il contient, est soumis à un traitement comprenant successivement une oxydation par un courant d'air entre 200° et 280°C, un traitement par de la vapeur d'eau sous pression et par de l'ammoniac, un lavage à l'eau chaude suivi d'un séchage au moyen d'un gaz à teneur majoritaire en hydrogène et enfin une réoxydation à l'air à environ 200°C.

Un inconvénient majeur du procédé précité réside dans le fait que la mise en oeuvre de la réactivation de l'absorbant chargé de mercaptans, par balayage réducteur en milieu hydrogène puis balayage oxydant par l'air conduit à une baisse d'activité assez rapide dudit absorbant et à la nécessité de régénérer cet absorbant en faisant appel à la technique complexe précitée peu adaptée à une mise en oeuvre industrielle du procédé. Un autre inconvénient dudit procédé est la nécéssité de disposer d'une source d'hydrogène sur le site de traitement pour effectuer le balayage réducteur de l'absorbant à régénérer.

On a maintenant trouvé des conditions particulières de mise en oeuvre de la régénération de l'absorbant ayant retenu les mercaptans, qui simplifient fortement la mise en oeuvre de la régénération et permettent de la réaliser facilement à l'échelle industrielle.

La régénération suivant l'invention de l'absorbant ayant retenu les mercaptans est d'une mise en oeuvre aisée et de plus elle conduit à un maintien dans le temps de l'activité de l'absorbant régénéré à une valeur élevée avec comme résultat un haut niveau de performance du procédé.

L'invention propose donc un procédé régénératif pour l'élimination des mercaptans contenus dans un gaz en faisant appel à un absorbant solide à base d'au moins un oxyde métallique actif, c'est-à-dire susceptible de retenir les mercaptans par réaction chimique, ledit procédé étant du type comportant une étape d'absorption au cours de laquelle on met en contact le gaz à traiter avec ledit absorbant, en opérant à des températures inférieures à 100°C, pour retenir les mercaptans sur l'absorbant et une étape de régénération et de refroidissement au cours de laquelle on balaie l'absorbant soumis à la régénération, dans une première phase, avec un gaz exempt d'oxygène libre et, dans une deuxième phase, en vue de réoxyder ledit absorbant et ainsi de le régénérer, avec un gaz renfermant de l'oxygène libre en effectuant cette seconde phase à des températures inférieures à 500°C jusqu'à complète réoxydation de l'absorbant et l'on refroidit l'absorbant régénéré jusqu'à une température appropriée pour sa réutilisation dans l'étape d'absorption, et se caractérisant en ce que l'on réalise la première phase du balayage de l'absorbant à régénérer en amenant ledit absorbant à une température comprise entre 250°C et 500°C, et de préférence entre 300°C et 450°C, par balayage avec un gaz inerte chaud et en poursuivant ledit balayage jusqu'à ce que l'absorbant ne renferme plus de composés soufrés et en ce que l'on effectue au moins la phase finale du refroidissement de l'absorbant réoxydé en employant un gaz inerte pour éliminer l'oxygène que peut contenir ledit absorbant réoxydé avant sa réutilisation dans l'étape d'absorption.

L'oxyde métallique actif ou les oxydes métalliques actifs, que peut renfermer l'absorbant, sont notamment choisis parmi les oxydes de métaux tels que cuivre, zinc, cadmium, mercure, fer, cobalt, argent, platine et plomb. Le procédé suivant l'invention s'applique avantageusement au traitement de gaz divers, notamment gaz naturels, gaz de synthèse ou même gaz résiduaires, renfermant, à titre d'impuretés, une faible quantité de mercaptans ayant de 1 à 8 atomes de carbone, et de préférence de 1 à 6 atomes de carbone, dans leur molécule, ces mercaptans étant des composés de formule RSH dans laquelle R désigne un radical hydrocarboné, notamment un radical alcoyle, en $C_1$ à $C_8$ et de préférence en $C_1$ à $C_6$.

Par le terme «faible quantité» on entend suivant l'invention une quantité globale de mercaptans représentant au plus 2% en volume du gaz à traiter.

L'absorbant à base d'oxyde métallique actif utilisé pour retenir les mercaptans est constitué avantageusement d'au moins un oxyde métallique actif associé à un support poreux inerte, ledit support étant en particulier un oxyde métallique poreux sans action sur les mercaptans et consiste de préférence en alumine. L'absorbant à base d'oxyde métallique actif présente avantageusement une surface spécifique, déterminée par la méthode d'absorption d'azote dite méthode BET, allant de 10 à 500 m²/g et de préférence de 100 à 300 m²/g.

L'absorbant peut être préparé en faisant appel à toute technique connue permettant de réaliser une association intime d'au moins un oxyde métallique actif et d'un support poreux inerte. On peut par exemple imprégner le support choisi avec un ou plusieurs sels générant l'oxyde métallique ou les oxydes métalliques actifs par calcination, puis sécher le support imprégné et calciner le produit séché à une température comprise entre 300°C et 600°C. On peut encore associer l'oxyde métallique actif ou les oxydes métalliques actifs au support par des techniques de coprécipitation suivies d'un séchage et d'une calcination dans l'intervalle de température précité ou encore par mélange de l'oxyde métallique actif ou des oxydes métalliques actifs et du support sous forme divisée.

La quantité totale d'oxyde métallique actif présente dans l'absorbant peut varier assez largement. Avantageusement cette quantité peut aller de 1 à 30% et de préférence de 5 à 20% en poids de l'absorbant.

Comme indiqué précédemment l'étape d'absorption, au cours de laquelle le gaz à traiter est mis en contact avec l'absorbant, est effectuée à des températures inférieures à 100°C, lesdites températures allant en particulier de 5 à 70°C et se situant de préférence au voisinage de la température ambiante, par exemple de 20°C à 50°C.

Les pressions utilisées dans l'étape d'absorption ne sont pas critiques. Généralement on met en oeuvre l'absorption à la pression à laquelle le gaz à traiter est disponible. Les pressions utilisées dans l'étape d'absorption peuvent aller de quelques bars, par exemple 2 à 5 bars (0,2 à 0,5 M Pa) pour le traitement d'un gaz résiduaire ou d'un gaz de synthèse, à plusieurs dizaines de bars, par exemple de 40 à 80 bars (4 à 8 M Pa) dans le cas du traitement d'un gaz naturel.

Le temps de contact du gaz à traiter avec l'absorbant doit avoir une valeur suffisante pour permettre une fixation quasi complète des mercaptans par l'absorbant. Des valeurs appropriées dudit temps de contact peuvent aller de 0,5 à 10 secondes et de préférence de 0,8 à 6 secondes TPN (température et pression normales), ce qui correspond à des valeurs de VVH (volume de gaz par volume de catalyseur et par heure) allant de 7200 à 360 h⁻¹ et de préférence de 4500 à 600 h⁻¹.

Au cours de l'étape d'absorption les mercaptans RSH contenus dans le gaz à traiter réagissent avec l'oxyde métallique actif présent dans l'absorbant et sont supposés être retenus par ledit absorbant en partie sous la forme de mercaptides métalliques et en partie sous la forme de disulfures R-S-S-R.

La première phase de l'étape de régénération et refroidissement du procédé suivant l'invention consiste à balayer l'absorbant ayant retenu les mercaptans, c'est-à-dire l'absorbant chargé de mercaptides métalliques et de disulfures, à l'aide d'un gaz chaud inerte pour amener ledit absorbant à une température comprise entre 250°C et 500°C, et de préférence entre 300°C et 450°C, et maintenir l'absorbant à cette température pendant une durée suffisante pour éliminer la quasi-totalité des composés soufrés qu'il renferme, de manière à obtenir un absorbant substantiellement exempt de produits soufrés.

Le gaz inerte utilisé pour effectuer ledit balayage peut être choisi parmi les divers gaz sans action sur les constituants de l'absorbant. Comme exemples de tels gaz on peut citer l'azote, les gaz rares, CO₂, les mélanges de tels gaz et même le gaz épuré résultant de l'application du procédé suivant l'invention à un gaz contenant des mercaptans.

Lorsque la phase de balayage de l'absorbant par le gaz inerte chaud est terminée, on fait passer un gaz contenant de l'oxygène libre au contact de l'absorbant débarassé des composés soufrés qu'il contenait, pendant une durée suffisante pour réoxyder complètement l'absorbant, c'est-à-dire pour reformer l'oxyde métallique actif.

La concentration en oxygène libre du gaz utilisé pour ladite réoxydation ne doit pas être trop élevée pour éviter une augmentation trop importante de la température de l'absorbant durant cette réoxydation. On obtient des résultats satisfaisants lorsque cette concentration est comprise entre 0,1 et 3% et plus particulièrement entre 0,5 et 1,5% en volume.

La gaz renfermant de l'oxygène libre peut être formé en ajoutant la quantité appropriée d'oxygène ou d'air au gaz inerte choisi pour effectuer la phase de balayage de l'absorbant ayant retenu les mercaptans.

Il est possible de réaliser simultanément d'une part la phase de mise en contact de l'absorbant débarrassé des composés soufrés, dont la température est comprise entre 250°C et 500°C avec le gaz contenant de l'oxygène libre en vue de réoxyder ledit absorbant et d'autre part la plus grande partie de la phase de refroidissement de l'absorbant en utilisant pour ladite mise en contact un gaz contenant de l'oxygène libre qui possède une température suffisamment basse et par exemple située aux environs de la température ambiante, et en poursuivant cette mise en contact jusqu'à ce que la température de l'absorbant ait été abaissée à la valeur désirée, après quoi on balaie l'absorbant régénéré refroidi au moyen d'un gaz inerte froid, choisi dans la même classe que le gaz inerte utilisé pour balayer l'absorbant à régénérer issu de l'étape d'absorption, jusqu'à ce que l'absorbant ne renferme plus d'oxygène absorbé.

On préfère cependant effectuer tout d'abord la mise en contact de l'absorbant débarrassé des composés soufrés avec le gaz renferment de l'oxygène libre en opérant à une température comprise entre 250°C et 500°C en particulier entre 300°C et 450°C, et notamment à une température substantiellement égale à celle à laquelle a été amené l'absorbant au cours de son balayage par le gaz inerte chaud, puis refroidir l'absorbant réoxydé jusqu'à la température choisie pour l'étape d'absorption par balayage à l'aide d'un gaz inerte froid tel que cité plus haut, ledit gaz ayant par exemple une température égale à la température ambiante ou voisine de cette dernière.

Les diverses phases de balayage de l'absorbant, qui sont réalisées au cours de l'étape de régénération et de refroidissement de l'absorbant, peuvent être effectuées sous des pressions qui, suivant les impératifs de rejet des effluents de ces balayages, peuvent se situer aux environs de la pression atmosphérique, par exemple de 1 à 5 bars (0,1 à 0,5 MPa), ou bien être sensiblement plus élevées, notamment 10 à 15 bars (1 à 1,5 MPa) ou plus, ou même atteindre des valeurs de l'ordre de 40 à 100 (4 à 10 MPa) bars.

Le procédé suivant l'invention peut être mis en oeuvre dans toute installation comportant au moins un réacteur, qui renferme un absorbant à base d'oxyde métallique actif et peut fonctionner alternativement en cycle d'absorption et en cycle de régénération et refroidissement comme indiqué précédemment. Avantageusement, pour obtenir une mise en oeuvre quasi-continue du procédé, on fait appel à une installation comportant au moins deux réacteurs disposés en parallèle et contenant un absorbant à base d'oxyde métallique actif, lesdits réacteurs étant agencés pour fontionner alternativement en cycle d'absorption et en cycle de régénération et refroidissement de telle sorte que l'un au moins des réacteurs soit un cycle de régénération et refroidissement tandis que le ou les autres réacteurs sont en cycle d'absorption.

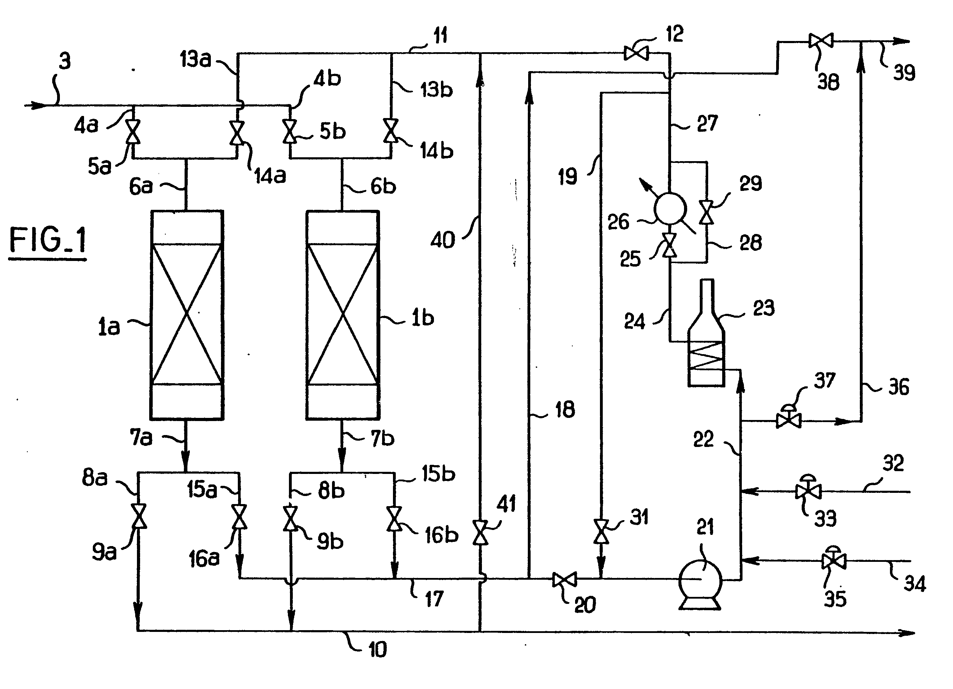

Sur les figures 1 à 3 du dessin annexé, on a représenté schématiquement trois exemples d'installations utilisables pour une mise en oeuvre quasi-continue du procédé suivant l'invention.

En se référant à la figure 1, l'installation comporte deux réacteurs 1a et 1b montés en parallèle et renfermant chacun une quantité appropriée d'un absorbant à base d'oxyde métallique actif. Chaque réacteur est pourvu d'un conduit d'entrée, respectivement 6a et 6b, et d'un conduit de sortie, respectivement 7a et 7b. Le conduit d'entrée 6a du réacteur 1a est connecté d'une part, par un conduit 4a muni d'une vanne 5a, à un conduit 3 d'amenée du gaz à traiter et d'autre part, par un conduit 13a muni d'une vanne 14a, à conduit 11 d'amenée d'un gaz de régénération pourvu d'une vanne 12. De même le conduit d'entrée 6b du réacteur 1b est connecté d'une part, par un conduit 4b pourvu d'une vanne 5b, au conduit 3 d'amenée du gaz à traiter et d'autre part, par un conduit 13b muni d'une vanne 14b, au conduit 11 d'amenée du gaz de régénération. Le conduit de sortie 7a du réacteur 1a est relié d'une part, par un conduit 8a muni d'une vanne 9a, à un conduit 10 d'évacuation du gaz épuré et d'autre part, par un conduit 15a muni d'une vanne 16a, à un conduit 17 de reprise de l'effluent de régénération connecté, à travers une vanne 20, à l'entrée d'une soufflante 21. De même le conduit de sortie 7b du réacteur 1b est relié d'une part, par un conduit 8b muni d'une vanne 9b, au conduit 10 d'évacuation du gaz épuré et d'autre part, par un conduit 15b muni d'une vanne 16b, au conduit 17 de reprise de l'effluent de régénération en amont de la vanne 20. Une dérivation 40 munie d'une vanne 41 relie le conduit 10 au conduit 11 en aval de la vanne 12 montée sur ce dernier conduit. De même un conduit 18 muni d'une vanne 38 est monté en dérivation sur le conduit 17 en amont de la vanne 20 et relie ledit conduit 17 au conduit 39 d'alimentation d'une torche.

La sortie de la soufflante 21 est connectée par un conduit 22 à l'entrée d'un four 23, dont la sortie est reliée, par un conduit 24 muni d'une vanne 25, à l'entrée d'un réfrigérant 26 dont la sortie est connectée, par un conduit 27, au conduit 11 en amont de la vanne 12 montée sur ce dernier conduit. Un conduit 28 muni d'une vanne 29 relie le conduit 24, en amont de la vanne 25, au conduit 27 et permet de court-circuiter le réfrigérant 26. Le conduit 27 est également connecté au conduit 17, entre la vanne 20 montée sur ce conduit et la soufflante 21, par un conduit 19 muni d'une vanne 31.

Un conduit 32 d'amenée d'azote, muni d'une vanne à débit variable 33, ainsi qu'un conduit 34 d'amenée d'air, pourvu d'une vanne à débit variable 35, sont connectés chacun au conduit 22 tandis que ledit conduit 22 est en outre relié, par un conduit 36 muni d'une vanne 37 à débit variable, au conduit 39 d'alimentation de la torche.

Le fonctionnement de cette installation peut être schématisé comme suit en se plaçant dans la situation où le réacteur 1a vient d'être mis en cycle d'absorption tandis que le réacteur 1b est isolé en vue d'être soumis au cycle de régénération.

Dans cette situation les vannes 5a, 9a, 29 et 31 sont ouvertes tandis que les vannes 5b, 9b, 12, 14a, 14b, 16a, 16b, 25, 20, 33, 35, 37, 38 et 41 sont fermées.

Le gaz à traiter arrive par le conduit 3, traverse le conduit 4a, dont la vanne 5a est ouverte, puis le conduit 6a et pénètre dans le réacteur 1a dans lequel il entre en contact avec l'absorbant à base d'oxyde métallique actif, ce dernier fixant les mercaptans contenus dans le gaz. Le gaz épuré sort du réacteur 1a par le conduit 7a, puis traverse le conduit 8a, dont la vanne 9a est ouverte, pour arriver dans le conduit 10 d'évacuation.

D'autre part un gaz inerte circule en circuit fermé depuis la soufflante 21 en passant par le conduit 22, le four 23, la partie du conduit 24 allant de la sortie du four 23 à la vanne 25 fermée, le conduit 28, dont la vanne 29 est ouverte, la partie du conduit 27 allant du conduit 28 au conduit 19, dont la vanne 31 est ouverte, et la partie du conduit 17 allant du conduit 19 à la soufflante 21.

A partir de ce moment le cycle de régénération de l'absorbant contenu dans le réacteur 1b est réalisé comme suit.

Dans un premier temps les vannes 16b et 38 sont ouvertes et le gaz contenu dans le réacteur 1b à la pression d'absorption s'échappe vers le conduit 39 d'alimentation de la torche en suivant le circuit formé par le conduit 7b, le conduit 15b, dont la vanne 16b est ouverte, la partie du conduit 17 allant du conduit 15b au conduit 18 et ledit conduit 18, dont la vanne 38 est ouverte. Cette opération permet de décomprimer le réacteur 1b.

A l'issue de la décompression du réacteur 1b, on ferme les vannes 31 et 38 et ouvre les vannes 12, 14b, 20, 33 et 37.

Le gaz inerte réchauffé par passage dans le four 23 traverse le conduit 11, dont la vanne 12 est ouverte, le conduit 13b, dont la vanne 14b est ouverte, et le conduit 6b et pénètre dans le réacteur 1b où il assure un balayage de l'absorbant chargé des produits soufrés formés pendant la phase d'absorption des mercaptans. Ledit balayage provoque une désoption des produits soufrés retenus par l'absorbant. Le gaz de balayage chargé des produits soufrés, désorbés sort du réacteur 1b par le conduit 7b, traverse le conduit 15b, dont la vanne 16b est ouverte, et passe dans le conduit 17, dont la vanne 20 est ouverte, jusqu'à la soufflante 21. Cette dernière renvoie le gaz de balayage qu'elle reçoit vers le four 23 à travers le conduit 22 et du four vers le conduit 11 comme indiqué plus haut. Une partie du gaz de balayage renfermant les produits soufrés désorbés passant dans le conduit 22 est dirigée par le conduit 36, avec un débit contrôlé par la vanne 37, vers le conduit d'alimentation de la torche 39. Un débit correspondant d'azote pur est injecté dans le conduit 22 par le conduit 32 à travers la vanne 33 à débit contrôlé.

La phase de balayage étant terminée, on ferme les vannes 33 et 37 et ouvre la vanne 35 pour admettre un débit contrôlé d'air dans le circuit de gaz passant par le réacteur 1b et l'on fait circuler ledit gaz, qui renferme alors une quantité contrôlée d'oxygène fonction du débit d'air introduit, jusqu'à réoxydation complète de l'absorbant, c'est-à-dire jusqu'à reformation de l'oxyde métallique actif.

A l'issue de cette réoxydation de l'absorbant, on ferme les vannes 29 et 35, ouvre la vanne 25 et arrête le four 23, ce qui conduit après une durée assez brève à envoyer dans le réacteur 1b un gaz inerte froid, qui assure le refroidissement de l'absorbant réoxydé.

Lorsque l'absorbant du réacteur 1b est refroidi à la température désirée, qui correspond généralement à celle utilisée pour la phase d'absorption, on ferme les vannes 12, 16b, 20 et 25 et ouvre les vannes 29, 31 et 41 de manière à isoler le réacteur 1b du circuit de gaz inerte de régénération et à faire passer dans ledit réacteur une partie du gaz épuré passant dans le conduit 10 pour assurer une recompression du réacteur 1b jusqu'à la pression d'absorption.

Lorsque la pression dans le réacteur 1b atteint une valeur sensiblement égale à la pression d'absorption, on ouvre les vannes 5b et 9b pour mettre le réacteur 1b en cycle d'absorption et dans le même temps ferme les vannes 5a, 9a, 14b et 41 pour isoler le réacteur 1a en vue de régénérer l'absorbant chargé de produits soufrés qu'il contient, ladite régénération étant réalisée comme indiqué plus haut dans le cas du réacteur 1b en remplaçant les vannes 5b, 9b, 14b et 16b par les vannes 5a, 9a, 14a et 16a.

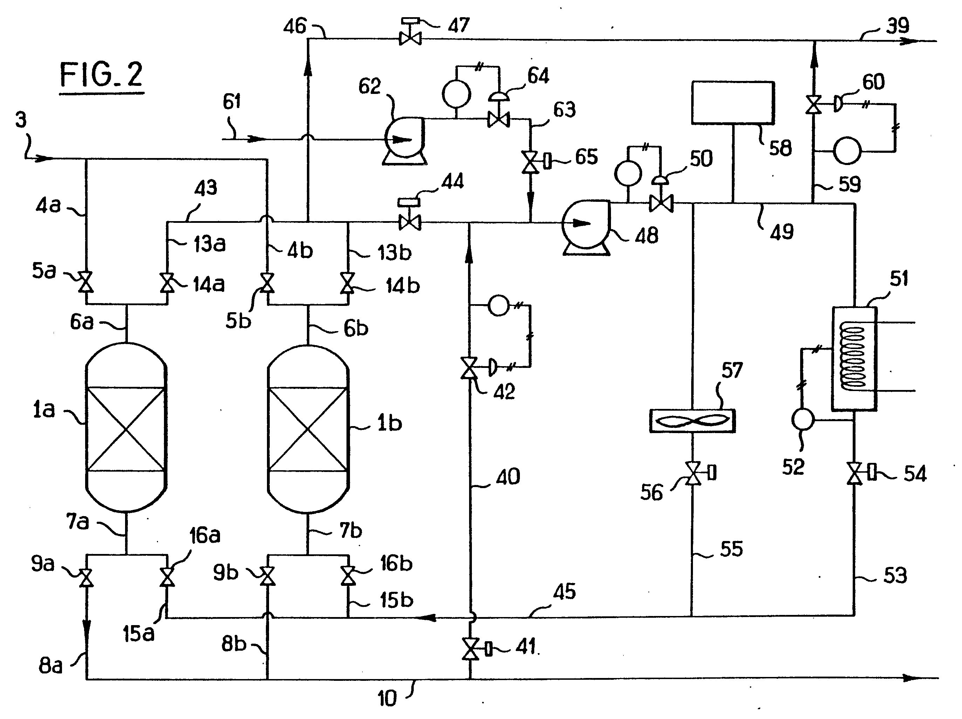

L'installation représentée sur la figure 2 comporte deux réacteurs 1a et 1b montés en parallèle et renfermant chacun une quantité appropriée d'un absorbant à base d'oxyde métallique actif. Chaque réacteur est pourvu à l'une de ses extrémités d'un premier conduit, respectivement 6a et 6b et à l'autre extrémité d'un second conduit, respectivement 7a et 7b. Le conduit 6a du réacteur 1a est connecté d'une part, par un conduit 4a muni d'une vanne 5a, à un conduit 3 d'amenée du gaz à traiter et d'autre part, par un conduit 13a muni d'une vanne 14a, à un conduit 43 de reprise de l'effluent de régénération pourvu d'une vanne 44. De même le conduit 6b du réacteur 1b est connecté d'une part, par un conduit 4b pourvu d'une vanne 5b, au conduit 3 d'amenée du gaz à traiter et d'autre part, par un conduit 13b muni d'une vanne 14b, au conduit 43. Le conduit 7a du réacteur 1a est relié d'une part, par un conduit 8a muni d'une vanne 9a, à un conduit 10 d'évacuation du gaz épuré et d'autre part, par un conduit 15a muni d'une vanne 16a, à un conduit 45 d'amenée d'un gaz de régénération. De même le conduit 7b du réacteur 1b est relié d'une part, par un conduit 8b muni d'une vanne 9b, au conduit 10 d'évacuation du gaz épuré et d'autre part, par un conduit 15b muni d'une vanne 16b, au conduit 45 d'amenée du gaz de régénération. Une dérivation 40 munie d'une vanne 41 et d'une vanne 42 de régulation de pression relie le conduit 10 au conduit 43 en aval de la vanne 44 montée sur ce dernier conduit. De même un conduit 46 muni d'une vanne 47 est monté en dérivation sur le conduit 43 en amont de la vanne 44 et relie ledit conduit 43 au conduit 39 d'alimentation d'une torche.

En aval de la vanne 44, le conduit 43 est relié à l'entrée d'une soufflante 48, dont la sortie est connectée par un conduit 49 muni d'une vanne 50 de régulation de débit à l'entrée d'un réchauffeur électrique 51 équipé d'un régulateur de température 52. La sortie du réchauffeur 51 est reliée, par un conduit 53 muni d'une vanne 54, au conduit 45 d'amenée du gaz de régénération aux réacteurs 1a et 1b. Un conduit 55 muni d'une vanne 56 et sur lequel est monté un aéroréfrigérant 57, relie le conduit 45 au conduit 49, en aval de la vanne 50 et permet de court-circuiter le réchauffeur 51. Un analyseur d'oxygène 58 est monté sur le conduit 49, en aval de la jonction de ce dernier avec le conduit 55, et en outre un conduit 59 équipé d'une vanne 60 de régulation de pression, relie le conduit 49, en aval de l'analyseur 58, au conduit 46, au point de jonction de ce dernier avec le conduit 39.

Un conduit 61 d'amenée d'air est connecté à l'entrée d'une soufflante 62, dont la sortie est reliée par un conduit 63, pourvu d'une vanne 64 de régulation de débit et d'une vanne 65, au conduit 43 en aval du point de jonction de ce conduit avec le conduit 40.

Le fonctionnement de cette installation peut être schématisé comme suit en se plaçant dans la situation où le réacteur 1a vient d'être mis en cycle d'absorption tandis que le réacteur 1b est isolé en vue d'être soumis au cycle de régénération.

Dans cette situation les vannes 5a, 9a, 41 et 54 sont ouvertes tandis que les vannes 5b, 9b, 14a, 14b, 16a, 16b, 44, 47, 56 et 65 sont fermées.

Le gaz à traiter arrive par le conduit 3, traverse le conduit 4a, dont la vanne 5a est ouverte, puis le conduit 6a et pénètre dans le réacteur 1a dans lequel il entre en contact avec l'absorbant à base d'oxyde métallique actif, ce dernier fixant les mercaptans contenus dans le gaz. Le gaz épuré sort du réacteur 1a par le conduit 7a, puis traverse le conduit 8a, dont la vanne 9a est ouverte, pour arriver dans le conduit 10 d'évacuation.

Le cycle de régénération de l'absorbant contenu dans le réacteur 1b est réalisé comme suit.

Dans un premier temps les vannes 14b et 47 sont ouvertes et le gaz contenu dans le réacteur 1b à la pression d'absorption s'échappe vers le conduit 39 d'alimentation de la torche en suivant le circuit formé par le conduit 6b, le conduit 13b, dont la vanne 14b est ouverte, et le conduit 46, dont la vanne 47 est ouverte. Cette opération permet de décomprimer le réacteur 1b.

A l'issue de la décompression du réacteur 1b, on ouvre les vannes 16b et 44.

La soufflante 48 aspire du gaz épuré par le conduit 40 et dirige ce gaz par le conduit 49 vers le réchauffeur 51, avec une pression contrôlée par la vanne 50 de régulation de pression.

Le gaz réchauffé par passage dans le réchauffeur 51 traverse le conduit 53, dont la vanne 54 est ouverte, le conduit 45 puis le conduit 15b dont la vanne 16b est ouverte, et le conduit 7b et pénètre dans le réacteur 1b où il assure un balayage de l'absorbant chargé des produits soufrés formés pendant la phase d'absorption des mercaptans. Ledit balayage provoque une désorption des produits soufrés retenus par l'absorbant. Le gaz de balayage chargé des produits soufrés désorbés sort du réacteur 1b par le conduit 6b, traverse le conduit 13b, dont la vanne 14b est ouverte, et passe en partie dans le conduit 46, dont la vanne 47 est ouverte, jusqu'au conduit 39 d'alimentation de la torche et en partie dans le conduit 43, dont la vanne 44 est ouverte, jusqu'à la soufflante 48. Cette dernière renvoie le gaz qu'elle reçoit vers le réchauffeur 51 comme indiqué plus haut. Au cours de ce balayage de l'absorbant, la pression du gaz mis en mouvement par la soufflante 48 est maintenue constant soit par appoint de gaz épuré prélevé sur le conduit 10, par le conduit 40, si la pression baisse, soit par un dégazage, par le conduit 59, vers le conduit 39 d'alimentation de la torche si la pression s'élève.

La phase de balayage étant terminée, on ferme les vannes 41 et 47 et ouvre la vanne 65 pour admettre un débit contrôlé d'air dans le circuit de gaz passant par le réacteur 1b et l'on fait circuler ledit gaz, qui renferme alors une quantité contrôlée d'oxygène fonction du débit d'air introduit, jusqu'à réoxydation complète de l'absorbant, c'est-à-dire jusqu'à reformation de l'oxyde métallique actif.

A l'issue de cette réoxydation de l'absorbant, on ferme les vannes 54 et 65, ouvre la vanne 56 et arrête le réchauffeur 51, ce qui conduit après une durée assez brève à envoyer dans le réacteur 1b un gaz inerte froid, qui assure le refroidissement de l'absorbant réoxydé.

Lorsque l'absorbant du réacteur 1b est refroidi à la température désirée, qui correspond généralement à celle utilisée pour la phase d'absorption, on ferme les vannes 14b et 44 et ouvre la vanne 41 de manière à faire passer dans ledit réacteur une partie du gaz épuré passant dans le conduit 10 pour assurer une recompression du réacteur 1b jusqu'à la pression d'absorption.

Lorsque la pression dans le réacteur 1b atteint une valeur sensiblement égale à la pression d'absorption, on ouvre les vannes 5b et 9b pour mettre le réacteur 1b en cycle d'absorption et dans le même temps on ferme les vannes 5a, 9a, 16b et 41 pour isoler le réacteur 1a en vue de régénérer l'absorbant chargé de produits soufrés qu'il contient, ladite régénération étant réalisée comme indiqué plus haut dans le cas du réacteur 1b en remplaçant les vannes 5b, 9b, 14b et 16b par les vannes 5a, 9a, 14a et 16a.

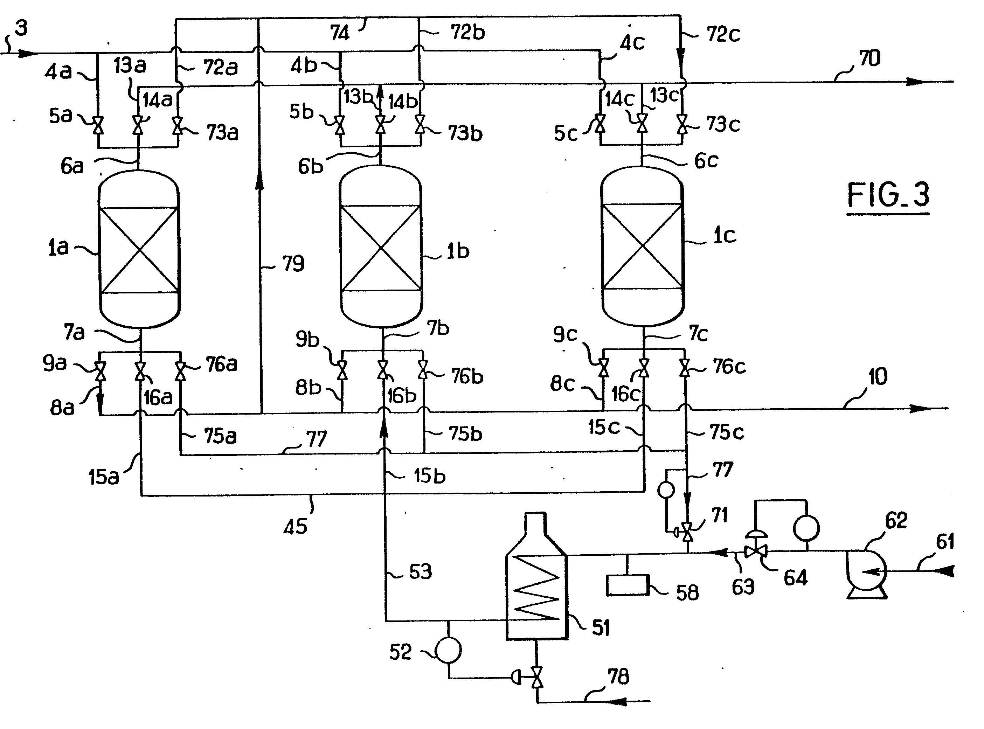

L'installation représentée sur la figure 3 comporte trois réacteurs 1a, 1b et 1c montés en parallèle et renfermant chacun une quantité appropriée d'un absorbant à base d'oxyde métallique actif. Chaque réacteur est pourvu à l'une de ses extrémités d'un premier conduit, respectivement 6a, 6b et 6c et à l'autre extrémité d'un second conduit, respectivement 7a, 7b et 7c. Le conduit 6a du réacteur 1a est connecté d'une part, par un conduit 4a muni d'une vanne 5a, à un conduit 3 d'amenée du gaz à traiter et d'autre part, par un conduit 13a muni d'une vanne 14a, à un conduit 70 de reprise de l'effluent de régénération, ledit conduit 6a étant en outre relié, par un conduit 72a muni d'une vanne 73a, à un conduit 74 d'amenée d'un gaz de refroidissement. De même le conduit 6b du réacteur 1b est connecté d'une part, par un conduit 4b pourvu d'une vanne 5b, au conduit 3 d'amenée du gaz à traiter et d'autre part, par un conduit 13b muni d'une vanne 14b, au conduit 70 de reprise de l'effluent de régénération et par un conduit 72b muni d'une vanne 73b au conduit 74 tandis que le conduit 6c du réacteur 1c est connecté, d'une part, par un conduit 4c muni d'une vanne 5c, au conduit 3 et d'autre part, par un conduit 13c pourvu d'une vanne 14c, au conduit 70 et par un conduit 72c muni d'une vanne 73c au conduit 74. Le conduit 7a du réacteur 1a est relié d'une part, par un conduit 8a muni d'une vanne 9a, à un conduit 10 d'évacuation du gaz épuré et d'autre part, par un conduit 15a muni d'une vanne 16a, à un conduit 45 d'amenée d'un gaz de régénération et par un conduit 75a muni d'une vanne 76a, à un conduit 77 de reprise de l'effluent de refroidissement. Ledit conduit 45 est connecté par un conduit 53 à la sortie d'un four 51, ledit four étant équipé d'un régulateur de température 52 ajustant le débit de combustible amené au four par un conduit 78, tandis que le conduit 77 est relié à l'entrée du four 51. De même le conduit 7b du réacteur 1b est relié d'une part, par un conduit 8b muni d'une vanne 9b, au conduit 10 d'évacuation du gaz épuré et d'autre part, par un conduit 15b muni d'une vanne 16b, au conduit 45 et par un conduit 75b muni d'une vanne 76b, au conduit 77 tandis que le conduit 7c du réacteur 1c est connecté d'une part au conduit 10, par un conduit 8c muni d'une vanne 9c, et d'autre part au conduit 45, par un conduit 15c muni d'une vanne 16c, et au conduit 77, par un conduit 75c muni d'une vanne 76c. Une dérivation 79 relie le conduit 10 au conduit 74.

Un conduit 61 d'amenée d'air est connecté à l'entrée d'une soufflante 62, dont la sortie est reliée, par un conduit 63 pourvu d'une vanne 64 de régulation de pression, au conduit 77 amenant l'effluent de refroidissement au four 51. Un analyseur d'oxygène 58 est monté sur le conduit 77 entre la jonction dudit conduit avec le conduit 63 et l'entrée du four 51, tandis qu'une vanne 71 de régulation de pression est montée sur le conduit 77 entre les jonctions dudit conduit avec respectivement le conduit 75c et le conduit 63.

Le fonctionnement de cette installation peut être schématisé comme suit en se plaçant dans la situation où le réacteur 1a vient d'être mis en cycle d'absorption tandis que le réacteur 1b vient d'être décomprimé pour être soumis au cycle de régénération et que le réacteur 1c est en phase de refroidissement.

Dans cette situation sont ouvertes les vannes 5a et 9a du réacteur 1a, les vannes 14b et 16b du réacteur 1b et les vannes 73c et 76c du réacteur 1c tandis que les autres vannes sont fermées.

Le gaz à traiter arrive par le conduit 3, traverse le conduit 4a, dont la vanne 5a est ouverte, puis le conduit 6a et pénètre dans le réacteur 1a dans lequel il entre en contact avec l'absorbant à base d'oxyde métallique actif, ce dernier fixant les mercaptans contenus dans le gaz. Le gaz épuré sort du réacteur 1a par le conduit 7a, puis traverse le conduit 8a, dont la vanne 9a est ouverte, pour arriver dans le conduit 10 d'évacuation.

Une partie du gaz épuré, qui est évacué par le conduit 10 et dont la température est voisine de la température ambiante, est prélevée par le conduit 79 et amenée par le conduit 74 et le conduit 72c, dont la vanne 73c est ouverte, au réacteur 1c qui vient d'être régénéré. Dans ce réacteur, ledit gaz entre en contact avec l'absorbant régénéré chaud et le refroidit.

L'effluent du réacteur 1c arrive dans le conduit 77, après avoir traversé le conduit 7c puis le conduit 75c dont la vanne 76c est ouverte, et, après détente par passage à travers la vanne 71, il pénètre ensuite dans le four 51, dans lequel ledit effluent est réchauffé à une température appropriée pour être utilisable pour la régénération. Le gaz réchauffé sortant du four 51 traverse le conduit 53, puis le conduit 15b, dont la vanne 16b est ouverte, et le conduit 7b et pénètre dans le réacteur 1b où il assure un balayage de l'absorbant chargé des produits soufrés formés pendant la phase d'absorption des mercaptans. Ledit balayage provoque une désorption des produits soufrés retenus par l'absorbant. Le gaz de balayage chargé des produits soufrés désorbés sort du réacteur 1b par le conduit 6b, traverse le conduit 13b, dont la vanne 14b est ouverte, et passe dans le conduit 70 pour être dirigé vers un incinérateur non représenté.

La désorption des produits soufrés étant terminée, on ouvre la vanne 64 pour admettre un débit contrôlé d'air dans le gaz arrivant au four 51 par le conduit 77 et passant après réchauffage par le réacteur 1b et l'on maintient l'injection d'air dans ledit gaz jusqu'à réoxydation complète de l'absorbant, c'est-à-dire jusqu'à reformation de l'oxyde métallique actif.

A l'issue de cette réoxydation de l'absorbant, on ferme la vanne 64 du conduit d'amenée d'air, puis les vannes 73c, 76c, 14b et 16b et on ouvre la vanne 73b. La pression dans le réacteur 1b augmente alors et lorsqu'elle a atteint une valeur proche de la pression du gaz épuré, on ferme les vannes 5a et 9a et ouvre les vannes 5c, 9c, 76b et la vanne 14a, puis la vanne 16a lorsque le réacteur 1a est décomprimé.

A la suite de ces diverses commutations de vannes, le réacteur 1c passe en phase d'absorption tandis que le réacteur 1b passe en phase de refroidissement et que le réacteur 1a passe en phase de régénération.

Les opérations se déroulent dans le temps de telle sorte qu'à chaque instant il y ait un réacteur en phase d'absorption, un réacteur en phase de régénération et un réacteur en phase de refroidissement et que chaque réacteur soit successivement en phase d'absorption, en phase de régénération et en phase de refroidissement.

L'invention est illustrée par les exemples suivants donnés à titre non limitatif.

*Exemple 1*

En opérant dans une installation analoque à celle schématisée sur la figure 1, du dessin annexé et fonctionnant comme décrit précédemment, on traitait un gaz naturel commercial renfermant 150 v.p.m. de méthylmercaptan, 50 v.p.m. de propylmercaptan, 5 p.p.m. d'$H_2S$ et 2000 p.p.m. d'hydrocarbures en $C_5$ à $C_8$.

Pour ce traitement chacun des deux réacteurs de l'installation renfermait 6,5 $m^3$ d'un absorbant se présentant en billes d'environ 5 mm de diamètre et consistant en une alumine imprégnée de 10% en poids d'oxyde cuivrique à titre d'oxyde métallique actif, ledit absorbant possédant une surface spécifique d'environ 190 $m^2$/g. La capacité dynamique de l'absorbant neuf correspondait à l'absorption d'une quantité de produits soufrés du type mercaptans représentant, comptée en soufre, 3,5 g par 100 g d'absorbant.

Les réacteurs fonctionnaient alternativement en cycle d'absorption et en cycle de régénération comme décrit plus haut en utilisant les conditions opératoires spécifiques ci-après.

*Cycle d'absorption:*

. Gaz à traiter arrivant par le
conduit 3 et entrant dans le
réacteur en absorption
  - Débit             : 41 667 $Nm^3$/heure
  - Pression      : 70 bars (7 MPa)

. Température d'absorption : 30°C

. Durée du cycle d'absorption                : 12 heures

*Cycle de régénération:*

. Phase de décompression
  - Durée            : 15 minutes
  - Vitesse de décom-    . 5 bars/minute
    pression           : (0,5 MPa/min)

. Balayage de l'absorbant à
régénérer par le gaz inerte
chaud
  - Débit du gaz de
    balayage        : 3850 $Nm^3$/heure
  - Température du gaz de
    balayage à la sortie du
    four 23         : 350°C

- Durée du balayage : 4 h 45 min
- Purge de gaz per la vanne 37 : 100 Nm³/heure
- Injection d'azote par la vanne : 100 Nm³/heure

. Réoxydation de l'absorbant
  - Durée : 2 heures
  - Température du gaz de réoxydation à la sortie du four 23 : 350°C
  - Concentration en oxygène dans le gaz de réoxydation (maintenue par injection contrôlée d'air par la vanne 35) : 1% en volume

. Refroidissement de l'absorbant réoxydé
  - Durée : 4 h 45 min
  - Débit de gaz de refroidissement : 3850 Nm³/heure
  - Température du gaz de refroidissement arrivant par le conduit 11 : 30°C

. Recompression du réacteur régénéré refroidi par le gaz épuré
  - Durée : 15 minutes
  - Vitesse de recompression : 5 bars/minute (0,5 MPa/min)

Le gaz épuré évacué par le conduit 10 renfermait une concentration globale en produits soufrés inférieure à 10 v.p.m.

Après 150 cycles d'absorption et de régénération la capacité dynamique de l'absorbant régénéré était encore pratiquement égale à celle de l'absorbant neuf.

*Exemple 2*

En opérant dans une installation analogue à celle schématisée sur la figure 2 du dessin annexé et fonctionnant comme décrit précédemment, on traitait un gaz naturel commercial renfermant 52 v.p.m. de méthylmercaptan, 18 v.p.m. de propylmercaptan, 5 p.p.m. d'$H_2S$ et 2000 p.p.m. d'hydrocarbures en $C_5$ à $C_8$.

Pour ce traitement chacun des deux réacteurs de l'installation renfermait 1,5 tonne d'un absorbant identique à celui utilisé dans l'exemple 1.

Les réacteurs fonctionnaient alternativement en cycle d'absorption et en cycle de régénération comme décrit en référence à la figure 2 en utilisant les conditions opératoires spécifiques ci-après.

*Cycle d'absorption:*

. Gaz à traiter arrivant par le conduit 3 et entrant dans le réacteur en absorption
  - Débit : 41 667 Nm³/heure
  - Pression : 70 bars (7 MPa)

. Température d'absorption : 30°C

. Durée du cycle d'absorption : 12 heures

*Cycle de régénération:*

. Phase de décompression
  - Durée : 15 minutes
  - Vitesse de décompression : 5 bars/minute (0,5 MPa/min)

. Balayage de l'absorbant à régénérer par le gaz inerte chaud
  - Débit du gaz de balayage : 1500 Nm³/heure
  - Température du gaz de balayage à la sortie du réchauffeur 51 : 350°C
  - Durée du balayage : 3 heures

. Réoxydation de l'absorbant
  - Durée : 2 heures
  - Température du gaz de réoxydation à la sortie réchauffeur 51 : 350°C
  - Concentration en oxygène dans le gaz de réoxydation (maintenue par injection contrôlée d'air par conduit 63) : 1,5% en volume

. Refroidissement de l'absorbant réoxydé
  - Durée : 6,5 heures
  - Débit de gaz de refroidissement : 750 Nm³/heure
  - Température du gaz de refroidissement arrivant par le conduit 55 : 30°C

. Recompression du réacteur régénéré refroidi par le gaz épuré
  - Durée : 15 minutes
  - Vitesse de recompression : 5 bars/minute (0,5 MPa/min)

Le gaz épuré évacué par le conduit 10 renfermait une concentration globale en soufre inférieure à 2 mg/Nm³.

*Exemple 3*

En opérant dans une installation analogue à celle schématisée sur la figure 3 du dessin annexé et fonctionnant comme décrit précédemment, on traitait un gaz naturel commercial renfermant 52 v.p.m. de méthylmercaptan, 18 v.p.m. de propylmercaptan, 5 p.p.m. d'$H_2S$ et 2000 p.p.m. d'hydrocarbures en $C_5$ à $C_8$.

Pour ce traitement chacun des trois réacteurs de l'installation renfermait 1,5 tonne d'un absorbant identique à celui utilisé dans l'exemple 1.

Les réacteurs fonctionnaient successivement en cycle d'absorption, en cycle de régénération et en cycle de refroidissement comme décrit en référence à la figure 3 en utilisant les conditions opératoires spécifiques ci-après.

*Cycle d'absorption:*

. Gaz à traiter arrivant par le
  conduit 3 et entrant dans le
  réacteur en absorption
  - Débit                          : 41 667 Nm$^3$/heure
  - Pression                       : 70 bars (7 MPa)

. Température d'absorption        : 30°C

. Durée du cycle d'absorption                             : 12 heures

*Cycle de régénération:*

. Phase de décompression
  - Durée                          : 1 heure
  - Vitesse de décom-              1,15 bar/minute
    pression                       (115 kPa/min)

. Balayage de l'absorbant à
  régénérer par le gaz de
  régénération chaud
  - Débit du gaz de
    balayage                       : 750 Nm$^3$/heure
  - Température du gaz de
    balayage à la sortie du
    four 51                        : 350°C
  - Durée du balayage              : 6 heures

. Réoxydation de l'absorbant
  - Durée                          : 4 heures
  - Température du gaz de
    réoxydation à la sortie
    du four 51                     : 350°C
  - Concentration en oxygène dans le gaz de
    réoxydation (maintenue
    par injection contrôlée
    d'air par le conduit 64)       : 1% en volume

. Recompression du réacteur régénéré refroidi par
  le gaz épuré
  - Durée                          : 1 heure
  - Vitesse de recom-              1,15 bar/minute
    pression                       (115 kPa/min)

*Cycle de refroidissement:*

. Refroidissement de l'absorbant réoxydé
  - Durée                          : 12 heures
  - Débit de gaz de refroi-
    dissement                      : 750 Nm$^3$/heure
  - Température du gaz de
    refroidissement arrivant
    par le conduit 74              : 30°C

Le gaz épuré evacué par le conduit 10 renfermait une concentration globale en soufre inférieure à 2 mg/Nm$^3$.

## Revendications

1. Procédé régénératif pour l'élimination des mercaptans contenus dans un gaz en faisant appel à un absorbant solide à base d'au moins un oxyde métallique actif, c'est-à-dire susceptible de retenir les mercaptans par réaction chimique, ledit procédé étant du type comportant une étape d'absorption au cours de laquelle on met en contact le gaz à traiter avec ledit absorbant, en opérant à des températures inférieures à 100°C, pour retenir les mercaptans sur l'absorbant et une étape de régénération et de refroidissement au cours de laquelle on balaie l'absorbant soumis à la régénération, dans une première phase, avec un gaz exempt d'oxygène libre et, dans une deuxième phase, en vue de réoxyder ledit absorbant et ainsi de le régénérer, avec un gaz renfermant de l'oxygène en effectuant cette seconde phase à des températures inférieures à 500°C jusqu'à complète réoxydation de l'absorbant et l'on refroidit l'absorbant régénéré jusqu'à une température appropriée pour sa réutilisation dans l'étape d'absorption, et se caractérisant en ce que l'on réalise la première phase du balayage de l'absorbant à régénérer en amenant ledit absorbant à une température comprise entre 250°C et 500°C par balayage avec un gaz inerte chaud et en poursuivant ledit balayage sensiblement à ladite température jusqu'à ce que l'absorbant ne renferme plus de composés soufrés et en ce que l'on effectue au moins la phase finale du refroidissement de l'absorbant réoxydé avec un gaz inerte pour éliminer l'oxygène que peut contenir ledit absorbant réoxydé avant sa réutilisation dans l'étape d'absorption.

2. Procédé suivant la revendication 1, caractérisé en ce que l'absorbant ayant retenu les mercaptans est amené à une température comprise entre 300°C et 450°C par balayage avec le gaz inerte chaud avant d'être mis en contact avec le gaz renfermant de l'oxygène libre.

3. Procédé suivant la revendication 1 ou 2, caractérisé en ce que le gaz à traiter renferme une quantité globale de mercaptans représentant au plus 2% en volume dudit gaz.

4. Procédé suivant l'une des revendications 1 à 3, caractérisé en ce que les mercaptans contenus dans le gaz à traiter répondent à la formule RSH, dans laquelle R désigne un radical hydrocarboné, notamment un radical alcoyle, en C$_1$ à C$_8$ et plus particulièrement en C$_1$ à C$_6$.

5. Procédé suivant l'une des revendications 1 à 4, caractérisé en ce que l'oxyde métallique actif ou les oxydes métalliques adtifs, que renferme l'absorbant, sont choisis parmi les oxydes de métaux cuivre, zinc, cadmium, mercure, fer, cobalt, argent, platine et plomb.

6. Procédé suivant l'une des revendications 1 à 5, caractérisé en ce que l'absorbant est constitué d'au moins un oxyde métallique actif associé à un support poreux inerte, ledit support étant en particulier un oxyde métallique poreux sans action sur les mercaptans comme l'alumine.

7. Procédé suivant la revendication 6, caractérisé en ce que l'absorbant a une surface spécifique, déterminée par la méthode BET, allant de 10 à 500 m$^2$/g et de préférence de 100 à 300 m$^2$/g.

8. Procédé suivant la revendication 6 ou 7, caractérisé en ce que la quantité totale d'oxyde métallique actif dans l'absorbant représente 1 à 30% et de préférence 5 à 20% du poids de l'absorbant.

9. Procédé suivant l'une des revendications 1 à 8, caractérisé en ce que l'étape d'absorption est mise en oeuvre à des températures allant de 5 à 70°C et de préférence de 20 à 50°C.

10. Procédé suivant l'une des revendications 1 à 9, caractérisé en ce que les pressions utilisées dans l'étape d'absorption vont de quelques bars, notamment 2 à 5 bars (0,2 à 0,5 MPa), à quelques dizaines de bars, notamment 40 à 80 (4 à 8 MPa) bars.

11. Procédé suivant l'une des revendications 1 à 10, caractérisé en ce que le temps de contact du gaz à traiter avec l'absorbant au cours de l'étape d'absorption va de 0,5 à 10 secondes et de préférence de 0,8 à 6 secondes.

12. Procédé suivant l'une des revendications 1 à 11, caractérisé en ce que le gaz inerte chaud utilisé pour balayer l'absorbant ayant retenu les mercaptans est choisi parmi l'azote, les gaz rares, $CO_2$, les mélanges de tels gaz et également le gaz épuré produit par le procédé.

13. Procédé suivant l'une des revendications 1 à 12, caractérisé en ce que le gaz renfermant de l'oxygène libre utilisé pour réoxyder l'absorbant a une concentration en oxygène libre comprise entre 0,1 et 3% et plus particulièrement entre 0,5 et 1,5% en volume.

14. Procédé suivant l'une des revendications 1 à 13, caractérisé en ce que la phase de mise en contact de l'absorbant ne renfermant plus de composés du soufre avec le gaz contenant de l'oxygène libre en vue de réoxyder l'absorbant et la plus grande partie de la phase de refroidissement de l'absorbant sont réalisées simultanément en utilisant pour ladite mise en contact un gaz renfermant de l'oxygène libre dont la température est suffisamment basse et se situe notamment aux environs de la température ambiante, après qui on balaie l'absorbant régénéré refroidi à l'aide d'un gaz inerte froid.

15. Procédé suivant l'une des revendications 1 à 13, caractérisé en ce que la mise en contact de l'absorbant débarrassé des produits soufrés avec le gaz contenant de l'oxygène libre est réalisé à une température comprise entre 250°C et 500°C et en particulier entre 300°C et 450°C, et notamment à une température substantiellement égale à celle à laquelle l'absorbant a été amené au cours de son balayage par le gaz inerte chaud, puis l'on refroidit l'absorbant réoxydé par balayage à l'aide d'un gaz inerte froid, ledit gaz ayant notamment une température égale à la température ambiante ou voisine de cette dernière.

**Patentansprüche**

1. Regeneratives Verfahren zur Abtrennung der in einem Gas enthaltenen Mercaptane unter Verwendung eines festen Absorbens auf der Basis wenigstens eines aktiven Metalloxids, d.h. eines solchen, das die Mercaptane durch chemische Reaktion zurückzuhalten vermag, wobei das besagte Verfahren eine Absorptionsstufe, während der das zu behandelnde Gas mit dem Absorbens in Kontakt gebracht wird, wobei bei Temperaturen unterhalb von 100°C gearbeitet wird, um die Mercaptane auf dem Absorbens festzuhalten, und eine Regenerations- und Abkühlungsstufe, während der das der Regeneration unterworfene Absorbens gespült wird, in einer ersten Phase mit einem keinen freien Sauerstoff enthaltenden Gas und in einer zweiten Phase, zur Reoxydation des Absorbens sowie zu seiner Regeneration, mit einem freien Sauerstoff enthaltenden Gas, wobei diese zweite Phase bei Temperaturen unterhalb 500°C bis zur vollständigen Reoxydation des Absorbens durchgeführt wird und das abgekühlte Absorbens bis zu einer für seine Wiederverwertung auf der Absorptionsstufe geeigneten Temperatur regeneriert wird, umfaßt, dadurch gekennzeichnet, daß man die erste Phase des Spülens des Absorbens zwecks Regeneration durchführt, indem man das Absorbens auf einer Temperatur zwischen 250 und 500°C durch Spülen mit einem heißen Inertgas bringt und die Spülung bei dieser Temperatur fortsetzt, bis das Absorbens keine Schwefelverbindungen mehr enthält, und daß man wenigstens die Endphase der Abkühlung des reoxydierten Absorbens mit einem Inertgas zur Abtrennung des Sauerstoffs, den das reoxydierte Absorbens enthalten kann, vor seiner Wiederverwertung auf der Absorptionsstufe durchführt.

2. Verfahren nach Anspruch 1, dadurch gekennzeichnet, daß das Absorbens, das die Mercaptane festhält, auf eine Temperatur zwischen 300 und 450°C durch Spülen mit dem heißen Inertgas gebracht wird, bevor es mit dem den freien Sauerstoff enthaltenden Gas in Kontakt gebracht wird.

3. Verfahren nach Anspruch 1 oder 2, dadurch gekennzeichnet, daß das zu behandelnde Gase eine Gesamtmenge an Mercaptanen von höchstens 2 Vol.-%, bezogen auf das Gas, enthält.

4. Verfahren nach einem der Ansprüche 1 bis 3, dadurch gekennzeichnet, daß die in dem zu behandelnden Gas enthaltenen Mercaptane der Formel RSH entsprechen, wobei R einen Kohlenwasserstoffrest, insbesondere einen Alkylrest mit 1 bis 8 C-Atomen und vorzugsweise mit 1 bis 6 C-Atomen, bedeutet.

5. Verfahren nach einem der Ansprüche 1 bis 4, dadurch gekennzeichnet, daß das aktive Metalloxid oder die aktiven Metalloxide, welche das Absorbens enthält, ausgewählt sind unter den Oxiden der Metalle Kupfer, Zink, Cadmium, Quecksilber, Eisen, Kobalt, Silber, Platin und Blei.

6. Verfahren nach einem der Ansprüche 1 bis 5, dadurch gekennzeichnet, daß das Absorbens gebildet ist aus wenigstens einem aktiven Metalloxid, das mit einem porösen inerten Träger verbunden ist, der insbesondere ein poröses Metalloxid ist, wie Tonerde, ohne Wirkung auf die Mercaptane.

7. Verfahren nach Anspruch 6, dadurch gekennzeichnet, daß das Absorbens eine nach der BET-Methode ermittelte spezifische Oberfläche von 10 bis 500 $m^2$/g, vorzugsweise 100 bis 300 $m^2$/g, aufweist,

8. Verfahren nach Anspruch 6 oder 7, dadurch gekennzeichnet, daß die Gesamtmenge an aktivem Metalloxid im Absorbens 1 bis 30 Gew.-%, vorzugsweise 5 bis 20 Gew.-% des Absorbens ausmacht.

9. Verfahren nach einem der Ansprüche 1 bis 8, dadurch gekennzeichnet, daß die Stufe der Absorption bei Temperaturen von 5 bis 70°C, vorzugsweise 20 bis 50°C, durchgeführt wird.

10. Verfahren nach einem der Ansprüche 1 bis 9, dadurch gekennzeichnet, daß die auf der Stufe der Absorption verwendeten Drücke von einigen bar, insbesondere 2 bis 5 bar (0,2 bis 0,5 MPa) bis zu einigen Dutzend bar, insbesondere 40 bis 80 bar (4 bis 8 MPa), reichen.

11. Verfahren nach einem der Ansprüche 1 bis 10, dadurch gekennzeichnet, daß die Dauer für die Kontaktierung des zu behandelnden Gases mit dem Absorbens während der Absorptionsstufe 0,5 bis 10 Sekunden, vorzugsweise 0,8 bis 6 Sekunden, beträgt.

12. Verfahren nach einem der Ansprüche 1 bis 11, dadurch gekennzeichnet, daß das zum Spülen des Absorbens, das die Mercaptane festhält, verwendete heiße Inertgas ausgewählt wird unter Stickstoff, Edelgasen, $CO_2$ sowie Gemischen dieser Gase und außerdem dem durch das Verfahren erzeugten Reingas.

13. Verfahren nach einem der Ansprüche 1 bis 12, dadurch gekennzeichnet, daß das freien Sauerstoff enthaltende Gas, das zur Reoxydation des Absorbens verwendet wird, eine Konzentration an freiem Sauerstoff von 0,1 bis 3 Vol.-% und vorzugsweise von 0,5 bis 1,5 Vol.-% aufweist.

14. Verfahren nach einem der Ansprüche 1 bis 13, dadurch gekennzeichnet, daß die Phase der Kontaktierung des keine Schwefelverbindungen mehr enthaltenden Absorbens mit dem freien Sauerstoff enthaltenden Gas zur Reoxydation des Absorbens und der Hauptteil der Phase der Abkühlung des Absorbens gleichzeitig durchgeführt werden, wobei man für diese Kontaktierung ein freien Sauerstoff enthaltendes Gas verwendet, dessen Temperatur ausreichend niedrig ist und insbesondere im Bereich der Umgebungstemperatur liegt, wonach man das abgekühlte regenerierte Absorbens mit Hilfe eines kalten Inertgases spült.

15. Verfahren nach einem der Ansprüche 1 bis 13, dadurch gekennzeichnet, daß die Kontaktierung des von Schwefelprodukten befreiten Absorbens mit dem freien Sauerstoff enthaltenden Gas bei einer Temperatur zwischen 250 und 500°C und insbesondere zwischen 300 und 450°C und ganz besonders bei einer Temperatur durchgeführt wird, die im wesentlichen der Temperatur entspricht, auf die das Absorbens während seines Spülens mit dem heißen Inertgas gebracht wurde, wonach man das reoxydierte Absorbens durch Spülen mit Hilfe eines kalten Inertgases abkühlt, das insbesondere eine Temperatur aufweist, die der Umgebungstemperatur entspricht oder in ihrer Nähe liegt.

## Claims

1. Regenerative process for the elimination of mercaptans contained in a gas, employing a solid absorbent on a basis of at least one active metallic oxide, that is to say capable of retaining the mercaptans by chemical reaction, the said method being of the type which comprises an absorption phase during the course of which the gas to be treated is brought into contact with the said absorbent, working at temperatures below 100°C, in order to retain the mercaptans on the absorbent, and a regeneration and cooling stage during the course of which the absorbent subjected to regeneration in a first phase is swept by a gas which is free from free oxygen and, in a second phase, with a view to reoxidising the said absorbent and thus regenerating it, with a gas containing free oxygen, this second phase being carried out at temperatures below 500°C until reoxidation of the absorbent is complete and the regenerated absorbent is cooled to a suitable temperature for it to be used again in the absorption stage, the process being characterised in that the first phase of sweeping the absorbent to be regenerated is carried out by bringing the said absorbent to a temperature between 250°C and 500°C by sweeping with a hot inert gas and pursuing the said sweeping substantially at the same temperature until such time as the absorbent no longer contains any sulphurous compounds and in that the final phase of cooling of the reoxidised absorbent is carried out with an inert gas to eliminate any oxygen which the said reoxidised absorbent may contain prior to its being used again in the absorption stage.

2. Process according to Claim 1, characterised in that the absorbent which has retained the mercaptans is brought to a temperature between 300°C and 450°C by sweeping with the hot inert gas prior to being brought into contact with the gas containing free oxygen.

3. Process according to Claim 1 or 2, characterised in that the gas to be treated contains a total quantity of mercaptans which represents at most 2% by volume of the said gas.

4. Process according to one of Claims 1 to 3, characterised in that the mercaptans contained in the gas to be treated satisfy the formula RSH, in which R designates a hydrocarbon radical, particularly an alkyl radical, in $C_1$ to $C_8$ and more particularly in $C_1$ to $C_6$.

5. Process according to one of Claims 1 to 4, characterised in that the active metallic oxide or active metallic oxides, contained in the absorbent, is/are chosen from among the oxides of the metals copper, zinc, cadmium, mercury, iron, cobalt, silver, platinum and lead.

6. Process according to one of Claims 1 to 5, characterised in that the absorbent consists of at least one active metallic oxide associated with an inert porous carrier, the said carrier being in particular a porous metallic oxide which has no action on the mercaptans, such as alumina.

7. Process according to Claim 6, characterised in that the absorbent has a specific surface area, ascertained by the BET method, ranging from 10 to 500 sq.m/g and preferably from 100 to 300 sq.m/g.

8. Process according to Claim 6 or 7, characterised in that the total quantity of active metallic oxide in the absorbent represents 1 to 30% and preferably 5 to 20% of the weight of the absorbent.

9. Process according to one of Claims 1 to 8, characterised in that the absorption stage is carried out at temperatures from 5 to 70°C and preferably 20 to 50°C.

10. Process according to one of Claims 1 to 9, characterised in that the pressures used in the absorption stage range from a few bars, in particular 2 to 5 bars (0.2 to 0.5 M Pa) to a few tens of bars, particularly 40 to 80 (4 to 8 M Pa) bars.

11. Process according to one of Claims 1 to 10, characterised in that the time during which the gas to be treated is in contact with the absorbent during the absorption stage ranges from 0.5 to 10 seconds and preferably from 0.8 to 6 seconds.

12. Process according to one of Claims 1 to 11, characterised in that the hot inert gas used for sweeping the absorbent which has retained the mercaptans is chosen from among the following: nitrogen, the rare gases, $CO_2$, mixtures of such gases and likewise the purified gas produced by the process.

13. Process according to one of Claims 1 to 12, characterised in that the gas containing free oxygen used for reoxidising the absorbent has a free oxygen concentration of between 0.1 and 3% and more particularly of between 0.5 and 1.5% by volume.

14. Process according to one of Claims 1 to 13, characterised in that the phase during which the absorbent which no longer contains any sulphur compounds is brought into contact with the gas containing free oxygen with a view to reoxidising the absorbent and the major part of the absorbent cooling phase are carried out simultaneously, the said contacting process being performed by use of a gas containing free oxygen, the temperature of which is sufficiently low, being in particular around ambient temperature, after which the cooled regenerated absorbent is swept by means of a cold inert gas.

15. Process according to one of Claims 1 to 13, characterised in that the absorbent which has been cleansed of sulphurous products is brought into contact with the gas containing free oxygen at a temperature comprised between 250°C and 500°C and in particular between 300°C and 450°C and in particular at a temperature which is substantially equal to that to which the absorbent was brought during the course of its being swept by the hot inert gas, after which the reoxidised absorbent is cooled by sweeping with a cold inert gas, the temperature of which is in particular equal or close to ambient temperature.

FIG.1

EP 0 221 942 B1

FIG.2

EP 0 221 942 B1

FIG.3